# EUROPÄISCHE PATENTSCHRIFT

(11) **EP 2 373 206 B1**
(45) Veröffentlichungstag und Bekanntmachung des Hinweises auf die Patenterteilung: **25.10.2017**
(21) Anmeldenummer: 09796632.9
(22) Anmeldetag: 22.12.2009
(51) Int. Cl.: A61B 1/12, A61L 2/28

(54) **TESTVORRICHTUNG FÜR ENDOSKOPWASCHMASCHINE**
TEST DEVICE FOR ENDOSCOPE WASHING MACHINE
DISPOSITIF DE TEST POUR APPAREIL DE LAVAGE D'ENDOSCOPES

(30) Priorität: 29.12.2008 DE 102008063273
(43) Veröffentlichungstag der Anmeldung: 12.10.2011
(73) Patentinhaber: Olympus Winter&Ibe GmbH, 22045 Hamburg (DE)
(72) Erfinder: ESCHBORN, Sascha, 22926 Ahrensburg (DE); WALDMANN, Jens, 22393 Hamburg (DE)
(74) Vertreter: Hausfeld, Norbert
(86) Internationale Anmeldenummer: PCT/EP2009/009223
(87) Internationale Veröffentlichungsnummer: WO 2010/075995

(56) Entgegenhaltungen:
- EP-A1- 0 709 056
- EP-A2- 1 779 769
- WO-A1-03/077960
- WO-A1-2008/019715
- US-A- 5 494 530
- US-A1- 2006 047 186

## Beschreibung

Die Erfindung betrifft eine Testvorrichtung der im Oberbegriff des Anspruches 1 genannten Art.

Medizinische Instrumente und Geräte, die bei Operationen mit dem Patientenkörper in Berührung kommen, werden entweder sterilverpackt zum Einmalgebrauch gekauft oder müssen nach jeder Benutzung gereinigt und desinfiziert bzw. sterilisiert werden.

Ein besonderer Problemfall sind flexible Endoskope. Diese kommen wegen ihres hohen Preises für Einmalverwendung nicht in Frage. Sie sind außerdem thermisch, chemisch und mechanisch empfindlich und müssen daher schonend behandelt werden. Zudem weisen flexible Endoskope Hohlräume und Kanäle auf, die einer besonderen Behandlung bedürfen.

Zum Reinigen und Desinfizieren flexibler Endoskope wurden daher Endoskopreinigungs- und -desinfektionsgeräte, im Folgenden "Waschmaschinen" genannt, entwickelt, die auch von der Anmelderin vertrieben werden. In solchen Waschmaschinen werden Endoskope in aufeinanderfolgenden Schritten mit Reinigungs- und Desinfektionsflüssigkeiten von außen und innen behandelt. Dabei werden die Endoskope mit Zugangsöffnungen an Anschlüsse der Waschmaschine angeschlossen, aus denen dem Endoskop Flüssigkeit unter Druck zugeführt wird. Die Flüssigkeit, also Reinigungs- oder Desinfektionsflüssigkeit, soll das Endoskop durchströmen. Dabei muss für eine gewisse Zeit eine ausreichende Durchströmung gewährleistet sein, um den gewünschten Reinigungs- und Desinfektionseffekt zu erzielen.

Die konstruktionsbedingt langen und dünnen Kanäle in flexiblen Endoskopen können bei oder nach einer Operation verstopfen, z.B. mit geronnenem Blut oder mit Geweberesten. Ist ein Kanal verstopft, so wird er nicht durchspült und somit unzureichend aufbereitet.

In solchen Waschmaschinen wird dies mit einer Prüfeinrichtung verhindert, die bei einem an ein Endoskop angeschlossenen Anschluss den Durchfluss prüft und z.B. mit einem Sollwert eines identischen, sauberen Endoskopes vergleicht. Registriert die Prüfeinrichtung eine Verstopfung, so wird dies angezeigt und das Endoskop muss länger gespült oder gegebenenfalls von Hand vorgereinigt und erneut mit der Waschmaschine behandelt werden.

Waschmaschinen mit solchen Prüfeinrichtungen sind aus DE 603 06 345 T2, EP 0 709 056 A1 und WO 03/077960 A1 bekannt.

Die ordnungsgemäße Funktion der Prüfeinrichtungen solcher Waschmaschinen ist von wesentlicher Bedeutung für den Erfolg der Reinigung der Endoskope. Die Prüfeinrichtungen müssen daher regelmäßig überprüft werden.

Die vorgenannten Schriften zeigen jedoch keine zum Testen von Prüfeinrichtungen geeignete Testvorrichtungen.

Aus dem Stand der Technik sind verschiedene Prüfeinrichtungen für Waschmaschinen bekannt.

Die DE 201 08 346 U1 zeigt ein Rohr, das an den Anschluss einer Waschmaschine anschließbar ist und in dessen Testraum Testverschmutzungen angebracht sind. Damit kann die Reinigungsleistung der Waschmaschine geprüft werden.

Die WO 2008/019715 A1 zeigt einen einem flexiblen Endoskop in der äußeren Form weitgehend angenäherten Endoskop-Dummy, der in Testräumen und auch auf der Außenseite Testverschmutzungen aufnehmen kann und mit dem auf diese Weise die Reinigungsleistung überprüfbar ist.

Diese beiden Schriften zeigen somit Testvorrichtungen, die nur die Reinigungsleistung, nicht aber die wichtigere ordnungsgemässe Funktion der Prüfeinrichtung testen können.

Der in der zweitgenannten Schrift gezeigte Endoskop-Dummy wird auch in dem Prospekt "BeliMed Infection Control" der Firma BeliMed AG, Zug, Schweiz, downloadbar von www.belimed.com, gezeigt. Auf Seite 3 im unteren Teil der rechten Spalte wird zu diesem Endoskop-Dummy ein "Verschlussadapter zur Simulation der Verblockung eines Kanals" angeboten. Wenn mit diesem ein Anschluss einer Waschmaschine blockiert wird, so kann damit überprüft werden, ob die Prüfeinrichtung der Waschmaschine einen verstopften Kanal korrekt erkennt. Dieser Verschlussadapter stellt somit eine gattungsgemäße Testvorrichtung dar.

Diese bekannte Testvorrichtung ist jedoch von äußerst einfacher Funktionsweise und erlaubt es nicht festzustellen, ob die Prüfeinrichtung wirklich bei einem realen Endoskop Fehler richtig erkennt.

Die Aufgabe der vorliegenden Erfindung besteht daher darin, eine gattungsgemäße Testvorrichtung zu schaffen, mit das Verhalten realer Endoskope nachbildbar ist.

Diese Aufgabe wird mit den Merkmalen des Kennzeichnungsteiles des Anspruches 1 gelöst.

Erfindungsgemäß ist der Testraum verstellbar ausgebildet. Das gibt die Möglichkeit, den Testraum hinsichtlich gewünschter Parameter einem realen Endoskop anzupassen. Man kann wie im tatsächlichen Betrieb der Waschmaschine unterschiedliche Endoskope nacheinander simulieren und erhält somit Testergebnisse, die verlässliche Rückschlüsse für den tatsächlichen Betrieb erlauben.

Flexible Endoskope weisen in ihrem Inneren neben durchgehenden Kanälen, die mit Flüssigkeit durchspült und überprüft werden, auch komplexe innere Hohlräume auf, die üblicherweise zusammenhängen und in denen empfindliche Einrichtungen, wie z.B. Elektronik, Optik und dergleichen, angeordnet sind. Diese Hohlräume liegen zwischen den Kanalwänden und den Außenwänden des Endoskopes und müssen sowohl zu den Kanälen als auch nach außen hin zuverlässig abgedichtet sein. Waschmaschinen weisen daher Dichtigkeitsprüfeinrichtungen auf, die an den Innenraum des Endoskopes einen Luftüber- oder -unterdruck anlegen und nach Absperren den Druckverlauf messen, um daraus die Dichtigkeit zu ermitteln. Auch diese Dichtigkeitsprüfeinrichtung der Waschmaschine sollte von der Testvorrichtung überprüft werden, wie dies z.B. auch bei der WO 2008/019715 A1 der Fall ist. Vorzugsweise sieht die Erfindung daher gemäß Anspruch 2 in der Testvorrichtung einen volumenverstellbaren Dichtigkeitstestraum vor, mit dem die Dichtigkeitsprüfeinrichtung der Waschmaschine überprüfbar ist. Die volumenverstellbare Ausbildung des Dichtigkeitsraumes ermöglicht dabei die exakte Nachbildung verschiedener realer Endoskope.
Vorzugsweise ist dabei gemäß Anspruch 3 der Dichtigkeitstestraum aus verbindbaren Teilräumen ausgebildet, so dass über einfache Schaltventile erforderliche Volumengrößen nachbildbar sind.

Zur Nachbildung der Kanäle eines Endoskopes ist der Testraum vorzugsweise gemäß Anspruch 4 als verstellbarer Testkanal ausgebildet. Dieser kann z.B. hinsichtlich seiner Länge oder seiner Durchgängigkeit verstellbar sein, um reale Endoskope nachzubilden.

Vorzugsweise ist der Testkanal gemäß Anspruch 5 aus über Schaltventile verbindbaren Teilkanälen ausgebildet. Damit können die komplexen Kanalsysteme unterschiedlicher Endoskope beliebig genau nachgebildet werden, so dass ein Testen der Prüfeinrichtung unter sehr realen Bedingungen ermöglicht wird.

Dabei ist vorzugsweise gemäß Anspruch 6 der Testkanal mit wenigstens einem einstellbaren Strömungswiderstand versehen. Damit lassen sich die Strömungswiderstände realer Endoskopkanäle genau nachbilden, so dass in komplexen Kanalsystemen die hydraulischen Verhältnisse exakt nachbildbar sind.

Vorzugsweise ist nach Anspruch 7 an den Testraum ein Druckmesser angeschlossen. Dieser kann z.B. bei der Überprüfung der Dichtigkeitsprüfeinrichtung dazu verwendet werden, den Druckmesser in der Prüfeinrichtung zu überprüfen. Der Druckmesser kann jedoch auch für verschiedene andere sicherheitsrelevante Tests verwendet werden, z.B. zum Überprüfen des Pumpdruckes der waschmaschinenseitigen Pumpe sowie zur Überprüfung von Sicherheitsdrucksensoren in der Waschmaschine, die bei gefährlichen Überdrücken abschalten sollen.

Vorzugsweise nach Anspruch 8 ist der Testraum über einen Durchflussmesser anschließbar. Damit wird unmittelbar der Durchflussmesser in dem angeschlossenen Anschluss der Prüfeinrichtung der Waschmaschine kontrolliert.

Die verschiedenen Anschlüsse der Waschmaschine für Flüssigkeit und Luft müssen an die entsprechenden Öffnungen des Endoskopes angeschlossen werden. Da die Öffnungen bei den Endoskopen je nach Typ oder Hersteller sehr unterschiedlich sind, werden Prüfadapter verwendet, die z.B. aus einfachen Schläuchen mit entsprechenden Kupplungsstücken an den Enden bestehen und die jeweils an einem Ende an einen Anschluss der Waschmaschine passen und am anderen Ende an eine anzuschließende Öffnung eines Endoskopes. Die erfindungsgemäße Testvorrichtung soll unterschiedliche Endoskope nachbilden und soll daher auch an den jeweils einem bestimmten Endoskop zugehörigen Prüfadapter passen. Die Testvorrichtung muss daher bei der Simulation eines Endoskopes auch die entsprechenden Anschlussöffnungen simulieren. Vorzugsweise wird das gemäß Anspruch 9 mit einem Testadapter gelöst, der einerseits an die Testöffnungen der Testvorrichtung passt und andererseits die Öffnungen des simulierten Endoskopes zur Verfügung stellt.

Vorzugsweise sind die Merkmale des Anspruches 10 vorgesehen. Dadurch kann aus einer in der Steuereinrichtung vorliegenden Tabelle, die zu einem zu simulierenden Endoskop die relevanten Konstruktionsdaten enthält, die Testvorrichtung derart eingestellt werden, dass dieses Endoskop genau nachgebildet wird. Durch Umschalten von Schaltventilen zwischen den Kanälen können z.B. Kanalverzweigungen nachgebildet werden. Die einzelnen Kanäle können durch Verstellen des Strömungswiderstandes an reale Kanäle angepasst werden. Auch der Innenraum des Endoskopes kann für die Überprüfung der Dichtigkeitsprüfeinrichtung in seinem Volumen verstellt werden.

Die Testvorrichtung soll, soweit möglich, ein reales Endoskop nachbilden und sollte daher auch zum Test anstelle eines Endoskopes in der Waschmaschine angeordnet sein. Dabei ist die Waschmaschine aber verschlossen, so dass die Testvorrichtung nicht mehr mit Anschlussleitungen von aussen versorgt werden kann. Vorzugsweise besitzt die Testvorrichtung daher gemäß Anspruch 11 eine eigene Energieversorgung.

In der Testvorrichtung werden Daten gewonnen, z.B. die des Druckmessers gemäß Anspruch 7 oder des Durchflussmessers gemäß Anspruch 8. Bei in der geschlossenen Waschmaschine angeordneter Testvorrichtung sind diese Daten nicht ohne Weiteres zugänglich. Vorzugsweise ist daher gemäß Anspruch 12 vorgesehen, dass die Testvorrichtung zur Speicherung oder gegebenenfalls zur Übertragung der Daten ausgebildet ist. Die gewonnenen Daten können also in der Testvorrichtung bis zum Entnehmen aus der Waschmaschine gespeichert sein, oder sie werden direkt während des Betriebes nach außen übertragen, z.B. über geeignete Funkverbindungen.

In den Zeichnungen ist die Erfindung beispielsweise und schematisch dargestellt. Es zeigen:
- Fig. 1: eine schematische Darstellung eines einfachen flexiblen Endoskopes mit angeschlossener Prüfeinrichtung einer Waschmaschine und
- Fig. 2: das schematische Diagramm einer erfindungsgemäßen Testvorrichtung.

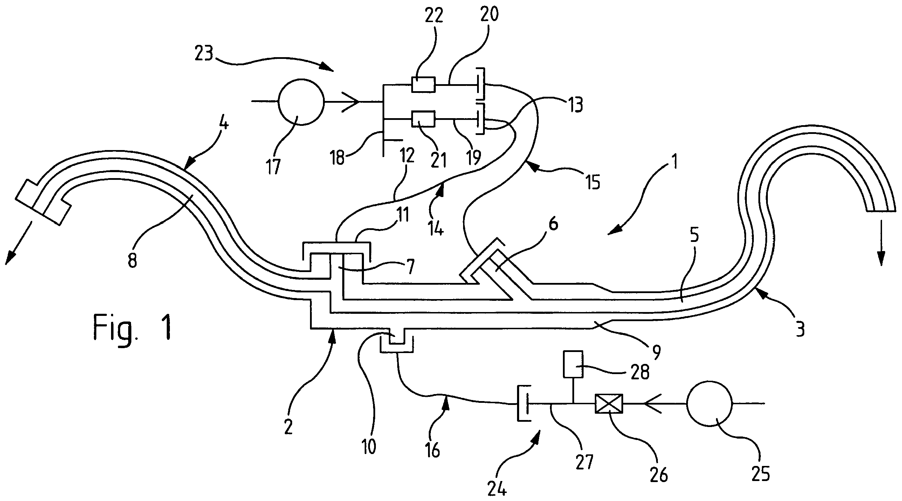

Fig. 1 zeigt in einer stark schematisierten Darstellung ein flexibles Endoskop 1, von dem nur die inneren und äußeren Wände als einfache Linie dargestellt sind. Das Endoskop 1 weist einen Hauptkörper 2 auf, von dem ein Einführungsschlauch 3 abgeht, welcher zum Einführen in den Körper, z.B. in den Darm eines Patienten, bestimmt ist. Außerdem geht vom Hauptkörper 2 ein Versorgungsschlauch 4 ab. Im Inneren des Endoskopes 1 ist ein Kanalsystem angeordnet mit einem Einführungskanal 5, der in der distalen Spitze des Einführungsschlauches 3 nach außen mündet. Der Einführungskanal 5 durchläuft auch den Hauptkörper 2. Eine Zugangsöffnung 6 ist nach außen hin offen und mündet in den Einführungskanal. Durch die Zugangsöffnung 6 können Biopsiezangen oder sonstige geeignet lange und flexible Arbeitsinstrumente in den Einführungskanal 5 vorgeschoben werden.

Der Einführungskanal 5 endet an einer Ventilöffnung 7, in der normalerweise ein Steuerventil angeordnet ist, das während der Reinigung aber ausgebaut ist. An der Ventilöffnung 7 geht vom Einführungskanal 5 ein Versorgungskanal 8 ab, der bis zum Ende des Versorgungsschlauches 4 läuft. Durch den Versorgungskanal 8 wird z.B. Spülflüssigkeit zugeführt und kann mit dem normalerweise in der Ventilöffnung 7 sitzenden Ventil gesteuert werden.

Um die Kanäle 5 und 8 herum, also zwischen diesen und der Außenwand des Endoskopes, ist ein Innenraum 9 ausgebildet, der sowohl gegenüber den flüssigkeitsführenden Kanälen als auch nach außen hin gut abgedichtet sein muss, da er in Fig. 1 nicht dargestellte empfindliche Einrichtungen aufnimmt, wie beispielsweise elektronische oder optische Einrichtungen. Der Innenraum 9 ist über eine Öffnung 10 von außen zugänglich. Diese wird üblicherweise dazu benutzt, während des Aufenthaltes in einer Waschmaschine den Innenraum 9 mit Druckluft zu beaufschlagen, damit durch eventuell vorhandene Lecks kein Wasser eindringen kann.

Fig. 1 zeigt das dargestellte Endoskop 1 im angeschlossenen Zustand, wie er beim Aufbereiten in einer Waschmaschine vorliegt. Die Ventilöffnung 7 ist über ein an diese angepasstes Kupplungsstück 11 eines Prüfadapters 14 angeschlossen, der aus dem Kupplungsstück 11, einem Schlauch 12 und einem Kupplungsstück 13 am anderen Ende des Schlauches besteht.

Ein weiterer Prüfadapter 15 ist mit einem passenden Kupplungsstück an die Zugangsöffnung 6 angeschlossen. Ein dritter Prüfadapter 16 ist entsprechend an die Öffnung 10 des Innenraumes 9 angeschlossen. Dabei sind die endoskopseitigen Kupplungsstücke der Prüfadapter passend für die Öffnungen des jeweiligen Endoskopes ausgebildet. Die anderen Kupplungsstücke passen zur Waschmaschine. Bei Anschluss eines anderen Endoskopes muss der Satz Prüfadapter gegebenenfalls gewechselt werden.

Die nicht näher dargestellte Waschmaschine weist eine Spülpumpe 17 auf, die in Pfeilrichtung Flüssigkeit, z.B. Wasser oder Desinfektionsflüssigkeit, in eine Verteilungsleitung 18 pumpt, von der ein erster Anschluss 19 über eine entsprechende Kupplung an den Prüfadapter 14 angeschlossen ist. Ein paralleler zweiter Anschluss 20 schließt an den Prüfadapter 15 an. Es kann also Flüssigkeit parallel in die Ventilöffnung 7 und die Zugangsöffnung 6 des Endoskopes 1 gepumpt werden. In den beiden Anschlüsse 19 und 20 befindet sich je ein Durchflussmesser 21, 22. Wie zeichnerisch mit einer Verzweigung angedeutet, können aus der Verteilungsleitung 18 noch weitere Anschlüsse mit Durchflussmessern abzweigen, die an weitere Öffnungen von komplexer aufgebauten Endoskopen anschließbar sind.

Die Durchflussmesser 21 und 22 sind an eine nicht dargestellte elektronische Einrichtung in der Waschmaschine angeschlossen, die die genauen Daten des angeschlossenen Endoskopes 1 kennt, insbesondere also dessen Kanalstruktur und Strömungswiderstände. Die Auswertung der mit den Durchflussmessern 21, 22 gemessene Strömungswerte erlaubt daher Aussagen über die Durchgängigkeit der Kanäle 5 und 8 und auch die Durchgängigkeit des Kanals 5 im Bereich zwischen den Öffnungen 6 und 7. Insbesondere kann natürlich eine teilweise oder völlige Blockade eines der Kanäle ermittelt werden.

Während des Aufbereitungsvorganges ist nicht nur die Kanalprüfeinrichtung 23, zu der der erste Anschluss 19 und der zweite Anschluss 20 gehören, angeschlossen, sondern auch eine Dichtigkeitsprüfeinrichtung 24, die an den Prüfadapter 16 und an den Innenraum 9 angekoppelt ist. Diese Dichtigkeitsprüfeinrichtung 24 führt dem Innenraum 9 ein Fluid in Form eines Gases zu, normalerweise Luft, und besitzt dazu eine Gaspumpe 25, die Gas in Pfeilrichtung durch ein Schaltventil 26 bis zur Kupplung mit dem Prüfadapter 16 pumpt. Zwischen Schaltventil 26 und Anschluss 27 ist ein Druckmesser 28 angeschlossen.

Die Dichtigkeitsprüfeinrichtung 24 beaufschlagt den Innenraum 9 des Endoskopes 1 mit Druck und sperrt dann das Schaltventil 26. Anschließend wird der Luft-Luftdruck mit dem Druckmesser 28 verfolgt. Aus der Druckabfallkurve kann die Größe einer vorhandenen Undichtigkeit berechnet werden.

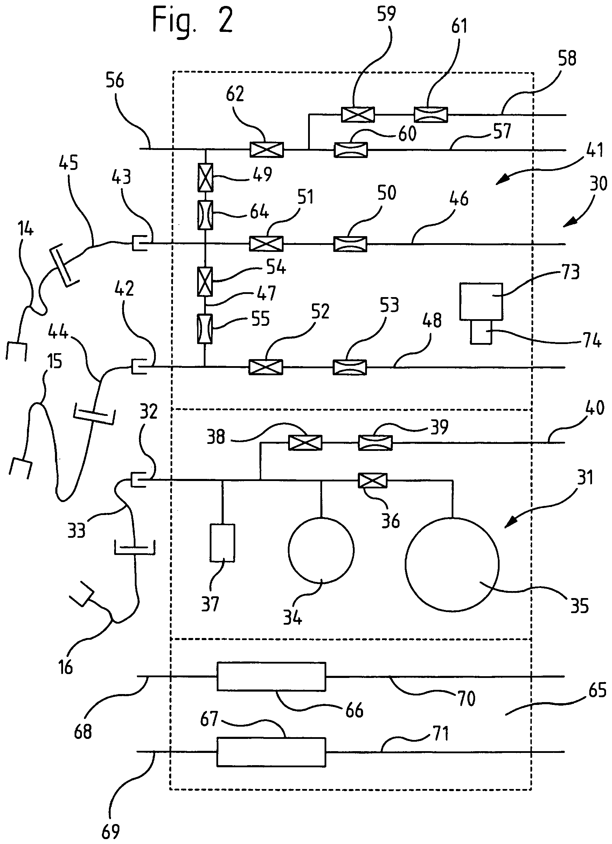

Zur Überprüfung der in Fig. 1 dargestellten, aus den Teilen 23 und 24 bestehenden Prüfeinrichtung der Waschmaschine dient die in Fig. 2 dargestellte Testvorrichtung 30.

Die Testvorrichtung 30 weist im dargestellten Ausführungsbeispiel drei mit gestrichelten Linien umrandete Abteilungen auf, die beispielsweise in einem gemeinsamen Gehäuse angeordnet sein können und jeweils eigene Testeinrichtungen aufweisen. Das Gehäuse ist vorzugsweise wasserdicht, damit die Testvorrichtung 30 zum Testen im Inneren einer Waschmaschine angeordnet werden kann.

Eine Dichtigkeitstesteinrichtung 31 weist eine stark schematisiert dargestellte Testöffnung 32 auf, die an den Anschluss 27 der Dichtigkeitsprüfeinrichtung 24 anzuschließen ist. Dieser Anschluss erfolgt über den Prüfadapter 16, der jedoch normalerweise nicht zur Testöffnung 32 passt. Deshalb ist zwischen dieser und dem Prüfadapter 16 ein zusätzlicher Testadapter 33 vorgesehen, der einerseits an die in Fig. 2 dargestellte Testvorrichtung 30 passt und andererseits die Öffnung 10 des Endoskopes 1 simuliert.

Die Dichtigkeitstesteinrichtung 31 soll den Innenraum 9 des Endoskopes 1 nachbilden, aber nicht nur diesen, sondern auch Innenräume anderer Endoskope. Dazu besitzt die Dichtigkeitstesteinrichtung 31 einen Dichtigkeitstestraum, der aus zwei Teilräumen 34 und 35 unterschiedlicher Größe besteht. Dabei ist der größere Teilraum 35 über ein Schaltventil 36 dem kleineren Teilraum 34 zuschaltbar. Der Teilraum 34 ist an die Testöffnung 32 angeschlossen. An diese ist außerdem ein Druckmesser 37 angeschlossen und über ein Schaltventil 38 und einen verstellbaren Strömungswiderstand 39 an eine nach außen führende Leitung 40. Die Dichtigkeitstesteinrichtung 31 kann mit den beiden Teilräumen 34 und 35 und gegebenenfalls weiteren zusätzlichen Teilräumen beliebige Innenraumgrößen von Endoskopen nachbilden. Durch Verstellen des Strömungswiderstandes 39 können beliebige Testlecks simuliert werden und mit dem Schaltventil 38 ein- und ausgeschaltet werden. Mit dem Druckmesser 37 kann unmittelbar der waschmaschinenseitig vorgesehene Druckmesser 28 geprüft werden.

Eine Kanaltesteinrichtung 41 weist zwei Testöffnungen 42 und 43 auf, die, wie auch die Testöffnung 32, in entsprechender Weise über Testadapter 44, 45 an die Prüfadapter 14 und 15 anschließbar sind. Mit diesen sind sie an die Anschlüsse 19 und 20 der Kanalprüfeinrichtung 23 anschließbar. Dabei sollen die Testöffnungen 42 und 43 der Zugangsöffnung 6 bzw. der Ventilöffnung 7 entsprechen, damit z.B. das in Fig. 1 dargestellte Endoskop 1 nachbildbar ist.

Die Kanaltesteinrichtung 41 weist eine Reihe von Kanälen auf, mit denen auch größere Kanalsysteme sehr komplexer Endoskope nachbildbar sind. Zur Nachbildung des Endoskopes 1 der Fig. 1 wird nur ein Teil davon benötigt.

Zur Nachbildung des Endoskopes 1 der Fig. 1 soll die Testöffnung 43, die an den Prüfadapter 14 angeschlossen ist, der Ventilöffnung 7 entsprechen. Von dort verläuft ein Kanal 46, der den Versorgungskanal 8 des Endoskopes 1 nachbildet, zu seinem freien Ende mit einem einen freien Ablauf gewährleistenden Abfluss.

Ein querverlaufender Kanal 47 verläuft zur Testöffnung 42, die der Zugangsöffnung 6 des Endoskopes 1 entspricht. Der Kanal 47 entspricht also dem Teil des Einführungskanales 5, der innerhalb des Hauptkörpers 2 des Endoskopes 1 zwischen den Öffnungen 6 und 7 liegt.

Von der Testöffnung 42 geht ein Kanal 48 ab, der ebenfalls zu einem freien Abfluss führt und der den von der Zugangsöffnung 6 bis zum distalen Ende des Einführungsschlauches 3 des Endoskopes 1 verlaufenden Teil des Einführungskanales 5 des Endoskopes 1 nachbildet. Weitere Teile des Kanalsystems der Kanaltesteinrichtung 41 werden zur Nachbildung des in Fig. 1 dargestellten Endoskopes 1 nicht benötigt. Dazu wird ein Schaltventil 49 geschlossen.

Zur Nachbildung der genauen Dimensionierung des Versorgungskanales 8 des Endoskopes 1 wird dessen Strömungswiderstand mit einem verstellbaren Strömungswiderstand 50 im Kanal 46 nachgebildet. Mit einem Schaltventil 51 kann der Kanal 46 völlig abgeschaltet werden, wenn er z.B. bei der Nachbildung eines anderen Endoskopes nicht benötigt wird.

Entsprechend ist auch der Kanal 48 verstellbar mit einem Schaltventil 52 und einem verstellbaren Strömungswiderstand 53 ausgerüstet. Der querverlaufende Kanal 47 zwischen den Testöffnungen 43 und 42 ist ebenfalls der Vollständigkeit halber mit Schaltventil 54 und verstellbarem Strömungswiderstand 55 ausgerüstet.

Durch Verstellung der einstellbaren Strömungswiderstände 50, 53 und 55 kann das Kanalsystem des Endoskopes 1 genau nachgebildet werden. Durch Schalten der Schaltventile können Kanäle völlig abgeschaltet werden. Dies wird insbesondere benötigt, wenn andere Endoskope nachgebildet werden. Es können zudem teilweise Verstopfungen nachgebildet werden durch Verstellen der verstellbaren Strömungswiderstände oder völlige Verstopfungen durch Schließen der Schaltventile. Die Kanalprüfeinrichtung 23 der Waschmaschine muss auf entsprechende Reaktionen überwacht werden.

Wie bereits erwähnt, kann die Kanalprüfeinrichtung 41 nicht nur das Endoskop 1 der Fig. 1 nachbilden, sondern auch andere Endoskope. Wird zusätzlich eine Testöffnung 56 verwendet mit einem nach außen führenden Kanal 57, so kann ein entsprechend ausgebildetes Endoskop nachgebildet werden. Ist in dem Endoskop parallel zu dem nach außen führenden Kanal 57 ein weiterer nach außen führender Kanal 58 vorgesehen, so kann dieser mit einem Schaltventil 59 zugeschaltet und abgeschaltet werden. Über einstellbare Strömungswiderstände 60 und 61 in den Kanälen 57 und 58 können deren Strömungswiderstände eingestellt werden. Bei Betätigung des Schaltventiles 59 wird der Kanal 58 zugeschaltet oder abgeschaltet. Die Kanalprüfeinrichtung 23 sollte das erkennen können.

Soll ein Endoskop simuliert werden, das dem der Fig. 1 entspricht, aber eine zusätzliche Öffnung aufweist, so wird die Testöffnung 56 verwendet, und die Kanäle 57 und 58 werden über ein Schaltventil 62 abgeschaltet. Das Schaltventil 49 wird geöffnet, um, gegebenenfalls unter Drosselung durch einen einstellbaren Strömungswiderstand 64 die Verbindung zu den Testöffnungen 43 und 42 herzustellen.

Die Darstellung der Kanaltesteinrichtung 41 in Fig. 2 soll nur beispielsweise die Möglichkeiten eines solchen Kanalsystems erläutern. Tatsächliche Testvorrichtungen können noch mehr Kanäle und Ventile aufweisen, um sehr komplexe Endoskope nachbilden zu können.

Neben den zwei besprochenen Abteilungen 31 und 41 weist die Testvorrichtung 30 noch eine dritte Abteilung 65 auf, die als Strömungstesteinrichtung dient. Sie enthält im Ausführungsbeispiel zwei Durchflussmesser 66 und 67, die an Testöffnungen 68 und 69 angeschlossen sind. An den anderen Enden sind die Durchflussmesser 66 und 67 mit den dargestellten Leitungen 70 und 71 nach außen angeschlossen.

Die Strömungstesteinrichtung 65 kann z.B. mit den Testöffnungen 68 und 69 und gegebenenfalls unter Verwendung von Adaptern an die Anschlüsse 19 und 20 der Kanalprüfeinrichtung 23 angeschlossen werden, so dass Flüssigkeit nacheinander durch die Durchflussmesser 22 und 66 bzw. 21 und 67 fließt, womit die waschmaschinenseitigen Durchflussmesser 22 und 21 unmittelbar überprüfbar sind.

Mit entsprechender Beschaltung durch zusätzliche Adapter oder durch innere Kanalverbindungen und Ventile können die Durchflussmesser 66 und 67 der Strömungstesteinrichtung 65 auch beispielsweise zwischen die Testöffnungen 42 und 43 der Kanaltesteinrichtung 41 und die davon abgehenden Adapter geschaltet werden, so dass bei Verwendung der Kanaltesteinrichtung 41 gleichzeitig auch die Strömungsmessung gestestet wird. Die Strömungstesteinrichtung 65 kann z.B. nur über einen Durchflussmesser verfügen oder auch mehr als die dargestellten zwei aufweisen. Ferner können in der Strömungstesteinrichtung 65 zusätzlich auch Druckmesser vorgesehen sein.

Wie Fig. 2 zeigt, ist an geeigneter Stelle in der Testvorrichtung 30 eine Steuereinrichtung 73 angeordnet, die mit nicht dargestellten Steuerleitungen an die darstellten Schaltventile und stellbaren Strömungswiderstände der Testvorrichtung 30 angeschlossen ist, um diese über entsprechende Steuersignale einzustellen. Informationen dazu kann die Steuereinrichtung 73 vorzugsweise aus einem eingebauten Speicher entnehmen, in dem die Daten unterschiedlicher Endoskope abgespeichert sind. Wird aus diesem Speicher z.B. das in Fig. 1 dargestellte Endoskop aufgerufen, so wird die Steuereinrichtung 73 bei der in Fig. 2 dargestellten Testeinrichtung 30 das Schaltventil 49 schließen und das Schaltventil 54 sowie die Schaltventile 51 und 52 öffnen. Die einstellbaren Strömungswiderstände 50, 53 und 55 würden auf die Werte des Endoskopes 1 gestellt. Die Steuereinrichtung 73 kann natürlich auch die Dichtigkeitstesteinrichtung 31 steuern.

Es sind weitere elektronische Einrichtungen vorsehbar, die in der Steuereinrichtung 73 integriert oder als gesonderte Einrichtungen ausgebildet sein können.

In der Testvorrichtung 30 fallen Daten an, z.B. bei Messungen des Druckmessers 37 und der Durchflussmesser 66 und 67. Auch können die Schalt- und Steuerzustände aller Schaltventile und Strömungswiderstände zur Protokollierung gespeichert werden. Dazu kann ein interner Speicher vorgesehen sein, der alle Messwerte solange speichert, bis der Testvorgang abgeschlossen ist und die Testvorrichtung 30 aus der Waschmaschine herausgenommen werden kann, um sie mit einer geeigneten Datenschnittstelle an einen Computer zum Abruf der Daten anzuschließen.

Es kann auch eine Datenübertragungseinrichtung vorgesehen sein, die die gewonnenen Daten laufend aus der Waschmaschine heraus überträgt, z.B. mit geeigneten drahtlosen Übertragungsverfahren.

Da die Testvorrichtung 30 längere Zeit im Inneren einer Waschmaschine arbeiten muss, wäre eine Kabelverbindung ungünstig. Das gilt nicht nur für die Datenübertragung sondern auch für die Energieversorgung. Aus diesem Grund ist eine in Fig. 2 dargestellte Batterie 74 vorgesehen, mit der die Steuereinrichtung 73 und auch die Schaltventile und stellbaren Strömungswiderstände versorgbar sind.

## Patentansprüche

1. Testvorrichtung (30) zum Testen der Prüfeinrichtung (23, 24) einer Waschmaschine für Endoskope (1), wobei die Prüfeinrichtung (23, 24) wenigstens einen Fluid unter Druck zuführenden, an eine Öffnung (6, 7, 10) eines Endoskopes (1) abgedichtet anschließbaren Anschluss (19, 20, 27) aufweist, der mit einem Durchflussmesser (21, 22) und/oder einem Druckmesser (28) versehen ist und wobei die Testvorrichtung (30) wenigstens einen über eine Testöffnung (32, 42, 43, 56, 68, 69) an den Anschluss (19, 20, 27) abgedichtet anschließbaren Testraum (34, 35, 70, 71, 46, 47, 48, 57, 58) aufweist, **dadurch gekennzeichnet, dass** der Testraum (34, 35, 70, 71, 46, 47, 48, 57, 58) zum Simulieren unterschiedlicher Endoskope verstellbar ausgebildet ist, wobei insbesondere ein Volumen und/oder ein Strömungswiderstand des Testraumes verstellbar ausgebildet ist.

2. Testvorrichtung nach Anspruch 1 für einen Gas über ein Schaltventil (26) und einen Druckmesser (28) zuführenden, die Gehäusedichtigkeit des Endoskopes (1) prüfenden Anschluss (27), **dadurch gekennzeichnet, dass** der Testraum als volumenverstellbarer Dichtigkeitstestraum (34, 35) ausgebildet ist.

3. Testvorrichtung nach Anspruch 2, **dadurch gekennzeichnet, dass** der Dichtigkeitstestraum aus mehreren über Schaltventile (36) verbindbaren Teilräumen (34, 35) besteht.

4. Testvorrichtung nach einem der vorhergehenden Ansprüche, für einen Flüssigkeit über einen Durchflussmesser (21, 22) zuführenden Anschluss (19, 20), **dadurch gekennzeichnet, dass** der Testraum mindestens einen von der Testöffnung (42, 43, 56) zu einem Abfluss führenden verstellbaren Testkanal (46, 47, 48, 57, 58) aufweist.

5. Testvorrichtung nach Anspruch 4, **dadurch gekennzeichnet, dass** der Testkanal aus über Schaltventile (49, 51, 52, 54, 59, 62) verbindbaren Teilkanälen (46, 47, 48, 57, 58) besteht.

6. Testvorrichtung nach einem der Ansprüche 4 oder 5, **dadurch gekennzeichnet, dass** der Testkanal wenigstens einen einstellbaren Strömungswiderstand (50, 53, 55, 60, 61, 62) aufweist.

7. Testvorrichtung nach einem der vorhergehenden Ansprüche, **dadurch gekennzeichnet, dass** der Testraum (34, 35) an einen Druckmesser (37) angeschlossen ist.

8. Testvorrichtung nach einem der vorhergehenden Ansprüche, **dadurch gekennzeichnet, dass** der Testraum (70, 71) über einen Durchflussmesser (66, 67) an die Testöffnung (68, 69) angeschlossen ist.

9. Testvorrichtung nach einem der vorhergehenden Ansprüche, **dadurch gekennzeichnet, dass** die Testöffnung (32, 42, 43) über einen Testadapter (33, 44, 45) an einen Prüfadapter (16, 15, 14) der Prüfeinrichtung (23, 24) anschließbar ist.

10. Testvorrichtung nach einem der vorhergehenden Ansprüche, **dadurch gekennzeichnet, dass** die schaltbaren und/oder stellbaren Einrichtungen (36, 38, 39, 49, 50, 51, 52, 53, 54, 55, 59, 60, 61, 62, 64) der Testvorrichtung (30) an eine Steuereinrichtung (73) angeschlossen sind, die zum Schalten oder Stellen anhand bereitgehaltener Daten verschiedener Endoskope ausgebildet ist.

11. Testvorrichtung nach einem der vorhergehenden Ansprüche, **dadurch gekennzeichnet, dass** die Testvorrichtung mit einer eigenen Energieversorgung (74) versehen ist.

12. Testvorrichtung nach einem der Ansprüche 7 oder 8, **dadurch gekennzeichnet, dass** die Testvorrichtung (30) zur Speicherung und/oder Übertragung der gemessenen Daten ausgebildet ist.

## Claims

1. Test device (30) for testing the checking facility (23, 24) of a washing machine for endoscopes (1), wherein the checking facility (23, 24) has at least one port (19, 20, 27) supplying fluid under pressure, sealed connectable to an opening (6, 7, 10) of an endoscope (1) which port has a flow meter (21, 22) and/or a pressure gauge (28) and wherein the test device (30) has at least one test space (34, 35, 70, 71, 46, 47, 48, 57, 58) connectible in a sealed manner to the port (19, 20, 27) via a test opening (32, 42, 43, 56, 68, 69), **characterized in that** the test space (34, 35, 70, 71, 46, 47, 48, 57, 58) is adjustably formed for simulating different endoscopes, wherein in particular a volume and/or a flow resistance of the test space are adjustably formed.

2. Test device according to Claim 1, for a port (27) supplying gas via an on-off valve (26) and a pressure gauge (28), checking tightness of the shell of the endoscope (1), **characterized in that** the test space is designed as a volume-adjustable leak test space (34, 35).

3. Test device according to Claim 2, **characterized in that** the leak test space is comprised of several subspaces (34, 35) connectible via on-off valves (36).

4. Test device according to any one of the preceding claims for a port (19, 20) supplying liquid via a flow meter (21, 22), **characterized in that** the test space has at least one adjustable test channel (46, 47, 48, 57, 58) leading from the test opening (42, 43, 56) to a drain.

5. Test device according to Claim 4, **characterized in that** the test channel consists of subchannels (46, 47, 48, 57, 58) connectible via on-off valves (49, 51, 52, 54, 59, 62).

6. Test device according to any of the claims 4 or 5, **characterized in that** the test channel has at least an adjustable flow resistance (50, 53, 55, 60, 61, 62).

7. Test device according to any one of the preceding claims, **characterized in that** the test space (34, 35) is connected to a pressure gauge (37).

8. Test device according to any one of the preceding claims, **characterized in that** the test space (70, 71) is connected to the test opening (68, 69) via a flow meter (66, 67).

9. Test device according to any one of the preceding claims, **characterized in that** the test opening (32, 42, 43) is connectible to a checking adapter (16, 15, 14) of the checking facility (23, 24) via a test adapter (33, 44, 45).

10. Test device according to any one of the preceding claims, **characterized in that** the switchable and/or adjustable facilities (36, 38, 39, 49, 50, 51, 52, 53, 54, 55, 59, 60, 61, 62, 64) of the test device (30) are connected to a control facility (73) adapted to switch or regulate by means of data kept ready of different endoscopes.

11. Test device according to any one of the preceding claims, **characterized in that** the test device is provided with its own power supply (74).

12. Test device according to any of the claims 7 or 8, **characterized in that** the test device (30) is adapted for storage and/or transmission of the measured data.

## Revendications

1. Dispositif de test (30) pour tester le dispositif d'essais (23, 24) pour appareil de lavage d'endoscopes (1), le dispositif d'essais (23, 24) présentant au moins un branchement (19, 20, 27) d'adduction de fluide sous pression pouvant être branché de façon étanche sur une ouverture (6, 7, 10) d'un endoscope (1), ce branchement d'adduction étant doté d'un débitmètre (21, 22) et/ou d'un manomètre (28), et le dispositif de test (30) présentant au moins un espace d'essai (34, 35, 70, 71, 46, 47, 48, 57, 58) pouvant être branché de façon étanche avec un orifice d'essai (32, 42, 43, 56, 68, 69) sur le branchement (19, 20, 27), **caractérisé en ce que** l'espace d'essai (34, 35, 70, 71, 46, 47, 48, 57, 58) est ajustable de façon à simuler différents endoscopes, en particulier **en ce qu'**un volume et/ou une résistance à l'écoulement de l'espace d'essai sont conçus de façon à pouvoir être ajustés.

2. Dispositif de test selon la revendication 1 pour un branchement (27) d'adduction de gaz par une soupape de commande (26) et un manomètre (28) pour vérifier l'étanchéité de l'enveloppe de l'endoscope (1), **caractérisé en ce que** l'espace d'essai est conçu sous forme d'espace d'essai d'étanchéité (34, 35) à volume ajustable.

3. Dispositif de test selon la revendication 2, **caractérisé en ce que** l'espace d'essai d'étanchéité est constitué de plusieurs espaces partiels (34, 35) pouvant être reliés par des soupapes de commande (36).

4. Dispositif de test selon l'une des revendications précédentes pour un branchement (19, 20) d'adduction d'un liquide par un débitmètre (21, 22), **caractérisé en ce que** l'espace d'essai présente au moins un canal d'essai (46, 47, 48, 57, 58) ajustable conduisant de l'orifice d'essai (42, 43, 56) à un écoulement.

5. Dispositif de test selon la revendication 4, **caractérisé en ce que** le canal d'essai est constitué de plusieurs canaux partiels (46, 47, 48, 57, 58) pouvant être reliés par des soupapes de commande (49, 51, 52, 54, 59, 62).

6. Dispositif de test selon l'une des revendications 4 ou 5, **caractérisé en ce que** le canal d'essai présente au moins une résistance à l'écoulement (50, 53, 55, 60, 61, 62) réglable.

7. Dispositif de test selon l'une des revendications précédentes, **caractérisé en ce que** l'espace d'essai (34, 35) est branché sur un manomètre (37).

8. Dispositif de test selon l'une des revendications précédentes, **caractérisé en ce que** l'espace d'essai (70, 71) est branché sur l'orifice d'essai (68, 69) par l'intermédiaire d'un débitmètre (66,67).

9. Dispositif de test selon l'une des revendications précédentes, **caractérisé en ce que** l'orifice d'essai (32, 42, 43) peut être branché par l'intermédiaire d'un adaptateur d'essai (33, 44, 45) sur un adaptateur de contrôle (16, 15, 14) du dispositif d'essais (23, 24).

10. Dispositif de test selon l'une des revendications précédentes, **caractérisé en ce que** les dispositifs commutables et/ou réglables (36, 38, 39, 49, 50, 51, 52, 53, 54, 55, 59, 60, 61, 62, 64) du dispositif de test (30) sont branchés sur un système de commande (73) conçu pour pouvoir commuter ou ajuster en fonction de données mises à disposition pour différents endoscopes.

11. Dispositif de test selon l'une des revendications précédentes, **caractérisé en ce que** le dispositif de test est équipé de sa propre alimentation en énergie (74).

12. Dispositif de test selon l'une des revendications 7 ou 8, **caractérisé en ce que** le dispositif de test (30) est conçu pour l'enregistrement et/ou la transmission des données mesurées.
